# EUROPEAN PATENT SPECIFICATION

(11) **EP 4 163 389 B1**
(45) Date of publication and mention of the grant of the patent: **26.08.2026**
(21) Application number: 21200842.9
(22) Date of filing: 05.10.2021
(51) Int. Cl.: C12Q 1/6806

(54) **METHOD FOR GENERATING A MARKER IN A BIOLOGICAL SAMPLE**
VERFAHREN ZUR ERZEUGUNG EINES MARKERS IN EINER BIOLOGISCHEN PROBE
PROCÉDÉ DE GÉNÉRATION D'UN MARQUEUR DANS UN ÉCHANTILLON BIOLOGIQUE

(43) Date of publication of application: 12.04.2023
(73) Proprietor: Leica Microsystems CMS GmbH, 35578 Wetzlar (DE)
(72) Inventor: Schlaudraff, Falk, 35510 Butzbach (DE)
(74) Representative: Schaumburg und Partner Patentanwälte mbB

(56) References cited:
- EP-A1- 3 686 273
- WO-A2-2004/045547
- US-A- 6 017 758
- ZHI PING ZHANG ET AL: "Photoregulation of protein plasmid expressionandusing BHQ caging group", CHINESE CHEMICAL LETTERS, vol. 22, no. 3, 22 December 2010 (2010-12-22), pages 338 - 341, XP028127785, ISSN: 1001-8417, [retrieved on 20101006], DOI: 10.1016/J.CCLET.2010.10.007
- MONROE W T ET AL: "Targeting expression with light using caged DNA", JOURNAL OF BIOLOGICAL CHEMISTRY, AMERICAN SOCIETY FOR BIOCHEMISTRY AND MOLECULAR BIOLOGY, US, vol. 274, no. 30, 23 July 1999 (1999-07-23), pages 20895 - 20900, XP002956517, ISSN: 0021-9258, DOI: 10.1074/JBC.274.30.20895
- SEBASTIAN HAUKE ET AL: "Specific protein labeling with caged fluorophores for dual-color imaging and super-resolution microscopy in living cells", CHEMICAL SCIENCE, vol. 8, no. 1, 1 January 2017 (2017-01-01), United Kingdom, pages 559 - 566, XP055601766, ISSN: 2041-6520, DOI: 10.1039/C6SC02088G
- ELLIS-DAVIES GRAHAM C R: "Caged compounds: photorelease technology for control of cellular chemistry and physiology", NATURE METHODS, vol. 4, no. 8, 1 August 2007 (2007-08-01), New York, pages 619 - 628, XP055807469, ISSN: 1548-7091, DOI: 10.1038/nmeth1072

## Description

### Technical field

The invention relates to a method for generating a marker in a biological sample by means of oligonucleotide constructs. In further aspects the invention relates to an oligonucleotide construct and a system for generating a marker in a biological sample.

### Background

Recent progress in the fields of cell culture research has led to the advent of 3D cell culture, which is based on cultivating cells in three dimensions, for example, in suspension culture (scaffold-free techniques) or embedded in hydrogels and/or extracellular matrices (scaffold-based techniques). Further, cultivation of a plurality of cell clusters such as spheroids is enabled by these techniques. It is often desirable to frequently analyse, in particular individually image, these cell clusters in order to track growth or viability, for example. These cell clusters may be individually imaged for analysis, for example, in a flow cell.

However, when culturing these cell clusters in suspension, (visually) identifying or repeatedly recognising specific cell clusters is difficult for a large plurality of cell clusters. This is especially the case for large numbers of uniform cell clusters, such as spheroids. Thus, it remains difficult to keep track of specific cell clusters within a plurality of cell clusters, especially when iteratively imaging these cell clusters over several time points.

### Summary

It is an object to provide a method and an oligonucleotide construct for generating a marker in a biological sample, which enable uniquely labelling the biological sample, in particularly, to subsequently identify the biological sample.

The aforementioned object is achieved by the subject-matter of the independent claims. Advantageous embodiments are defined in the dependent claims and the following description.

In a first aspect a method is provided for generating a marker in a biological sample comprising a plurality of cells by means of oligonucleotide constructs. The method comprises the following steps: introducing at least a plurality of first oligonucleotide constructs into the biological sample, wherein the first oligonucleotide constructs comprise a first promoter, a first nucleic acid sequence encoding a first fluorescent protein, and a first photoremovable cage molecule; and exposing, in particular scanning, at least a first region of the biological sample with a first spatially constrained, in particular focused, light beam to form uncaged first oligonucleotide constructs in order to enable synthesis of first fluorescent proteins from the first nucleic acid sequence and generate at least a part of the marker in the first region of the biological sample. The biological sample may be in the form of a spheroid and, in particular, comprise living cells. In other words, the uncaging of the oligonucleotide constructs enables expression of the nucleic acid sequence and generation of the fluorescent protein in the cell or cells of the biological sample within the first region. This enables generating a marker in form of at least the first region in order to identify the marked biological sample based on the particular marker.

It is preferred, that the biological sample is imaged prior to the exposing step in order to generate an image of the biological sample, in particular to identify at least the first region in the image data, and/or wherein the biological sample is imaged after the exposing step in order to generate an image of the biological sample with the marker. By imaging the biological sample prior to generating the marker a suitable area for the region of the biological sample can be identified in the image. Further, coordinates of the region may be determined based on the position of the biological sample relative to an optical device configured to image the biological sample, enabling exposure of the region with high accuracy. In addition, the image of the biological sample prior to the exposure step may act as a baseline to compare further images to. In case images are taken after the exposure step, these images may be used to check successful generation of the marker. Further, these images after the exposure step may be compared to the baseline image.

Preferably, the step of introducing the oligonucleotide constructs is performed by transfection, in particular by lipofection or electroporation. This enables particularly efficient introduction of the oligonucleotide constructs into the biological sample.

In a preferred embodiment, a plurality of second oligonucleotide constructs is introduced into the biological sample, wherein the second oligonucleotide constructs comprise a second promoter, a second nucleic acid sequence encoding a second fluorescent protein, and a second photoremovable cage molecule. This enables generating a large number of distinguishable and unique markers.

In a particularly preferred embodiment, a second region of the biological sample is exposed with a second spatially constrained, in particular focused, light beam to form uncaged second oligonucleotide constructs in order to enable synthesis of second fluorescent proteins from the second nucleic acid sequence and generate at least a part of the marker in the second region of the biological sample. This enables the selective uncaging of the first and the second oligonucleotide constructs.

It is particularly preferred, that the first spatially constrained light beam and the second spatially constrained light beam differ in at least one light parameter, wherein said at least one light parameter comprises an uncaging wavelength, a predetermined pulse length or a light intensity. This enables the selective uncaging of the first and the second oligonucleotide constructs.

Preferably, the first fluorescent protein and the second fluorescent protein have different fluorescent emission wavelengths. This enables optically distinguishing between the first regions and the second regions.

In a preferred embodiment, the spatially constrained light beams are generated by means of an optical device, in particular, by a widefield fluorescence microscope, an epi-fluorescence microscope, a light-sheet microscope, a confocal microscope, or a multiphoton microscope. The optical device may further be used for imaging the biological sample, for example, in a flow cell. This enables exposing the regions of the biological sample with the light beams with high precision and thus generate the marker with high precision.

Preferably, the regions of the biological sample have one of the following shapes: circular, ovoid, linear, polygon, number, letter, a linear or curved dashed and/or point like structure (comparable to a Morse code), or the shape of a single cell of the biological sample. Such shapes or structures may be generated by a scanning microscope which is adapted to, for example, scan a spatially constrained - in particular a focused - light distribution along a two- or three-dimensional path that generates such a shape or structure. This enables generating a larger number of unique regions.

In a further aspect, an oligonucleotide construct is provided for generating a marker in a biological sample, in particular cellular samples. The oligonucleotide construct comprises a promoter, a nucleic acid sequence encoding a fluorescent protein, and at least one photoremovable cage molecule, photoremovable from the oligonucleotide construct by a spatially constrained, in particular focused, light beam. The removing of the cage molecule and thus generation of an uncaged oligonucleotide construct enables expression of the nucleic acid sequence and thus generation of the fluorescent protein.

Preferably, the oligonucleotide construct comprises RNA nucleotides, DNA nucleotides and/or locked nucleic acid (LNA) nucleotides. This enables flexible manufacture of the oligonucleotide construct.

It is preferred, that the oligonucleotide construct is linear or circular. This enables varying the half-life of the oligonucleotide construct, with circular oligonucleotides generally being more stable and having a longer half-life.

Preferably, the photoremovable cage molecules are attached to the promoter. This enables particularly effective caging of the oligonucleotide construct.

The following embodiments relating to a system for generating a marker in a biological sample, in particular cellular samples, are not encompassed by the wording of the claims. The system comprises: at least one first oligonucleotide construct comprising a first promoter, a first nucleic acid sequence encoding a first fluorescent protein, and a first photoremovable cage molecule; wherein the first photoremovable cage molecule is photoremovable from the first oligonucleotide construct by a first spatially constrained, in particular focused, light beam; and an optical device configured to provide a first spatially constrained light beam and to generate the marker in the biological sample. The selective removal of the first cage molecule and thus selective generation of a respective uncaged oligonucleotide construct enables selective expression of the respective nucleic acid sequence and thus generation
of the corresponding fluorescent protein only in areas exposed to corresponding light beam. Further, markers may be generated by the system in more than the one biological sample.

Preferably, the system further comprises at least one second oligonucleotide construct comprising a second promoter, a second nucleic acid sequence encoding a second fluorescent protein, and a second photoremovable cage molecule; wherein the second photoremovable cage molecule is photoremovable from the second oligonucleotide construct by a second spatially constrained, in particular focused, light beam, and wherein the optical device is further configured to provide a second spatially constrained light beam to generate the marker in the biological sample. The selective removal of the first and second cage molecules and thus selective generation of uncaged oligonucleotide constructs enables selective expression of the respective nucleic acid sequence and thus generation of the corresponding fluorescent protein only in areas exposed to corresponding light beam.

In a particularly preferred embodiment, the optical device is configured to expose at least a first region of the biological sample to the first light beam and at least a second region of the biological sample to the second light beam. The light beams uncage the respective oligonucleotide constructs in order to enable synthesis of the corresponding fluorescent proteins and generate at least a part of the marker in the respective region of the biological sample.

In a preferred embodiment, the first fluorescent protein and the second fluorescent protein have different fluorescent emission wavelengths. This enables optically distinguishing between the first regions and the second regions.

Preferably, the first spatially constrained light beam and the second spatially constrained light beam differ in at least one light parameter, wherein said at least one light parameter comprises an uncaging wavelength, a predetermined pulse length, or a light intensity. This enables the selective uncaging of the first and the second oligonucleotide constructs.

It is preferred, that the first promoter and the second promoter have the same nucleotide sequence, or that the first promoter and the second promoter have different nucleotide sequences. This enables matching the promoter activity to the fluorescence emission intensity of the respective fluorescent protein.

### Short Description of the Figures

Hereinafter, specific embodiments are described referring to the drawings, wherein:
- Fig. 1: shows a schematic view of an oligonucleotide construct for generating a marker in a biological sample,
- Fig. 2: shows a flow chart of a method for generating a marker in a biological sample, and
- Fig. 3: shows three biological samples with markers.

### Detailed Description

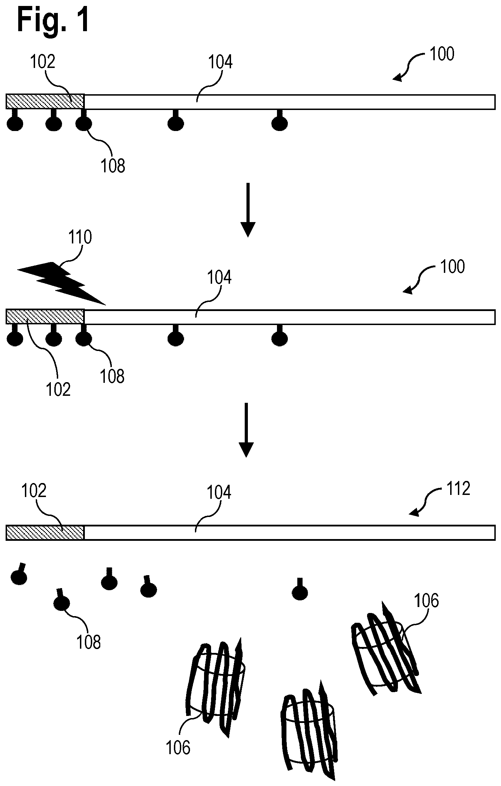

Figure 1 shows an oligonucleotide construct 100 for generating a marker in a biological sample. The oligonucleotide construct 100 is a single stranded, or alternatively double stranded, deoxyribonucleic acid (DNA) molecule comprising a promoter region 102 and a fluorescent protein region 104. The fluorescent protein region 104 is a nucleic acid sequence that encodes a first fluorescent protein 106. The promoter 102 is a nucleic acid sequence that acts as the starting point for transcription and/or translation, in order to synthesise the fluorescent protein 106 from the fluorescent protein region 104. The promoter is at a 5' end of the oligonucleotides construct 100. The construct 100 may additionally or alternatively be a ribonucleic acid (RNA) and/or a locked nucleic acid (LNA).

The oligonucleotide construct 100 further comprises at least one photoremovable cage molecule 108, preferably bound to the promoter region 102. The photoremovable cage molecule 108 is a photolabile protecting group and cleavable from the oligonucleotide construct 100 by light 110 at a predetermined wavelength. In particular, the cage molecule 108 may be covalently bound to a nucleobase of the promoter region 102, with the light 110 causing the covalent bond to break and release the cage molecule. The bound cage molecule 108 prevents, for example, the binding of enzymes to the promoter region 102. This prevents transcription and/or translation of the oligonucleotide construct 100, and therefore the synthesis of the fluorescent protein 106 from the fluorescent protein region 104. Regularly, the construct 100 comprises a plurality of cage molecules 108.

Figure 1 further shows the cage molecules 108 removed from the oligonucleotide construct 100 to form an unprotected oligonucleotide construct 112. In the presence of suitable enzymes, such as polymerases and/or ribosomes, and additional elements such as nucleotides and/or transfer RNA molecules, the fluorescent protein 106 may be synthesised from the unprotected oligonucleotide construct 112.

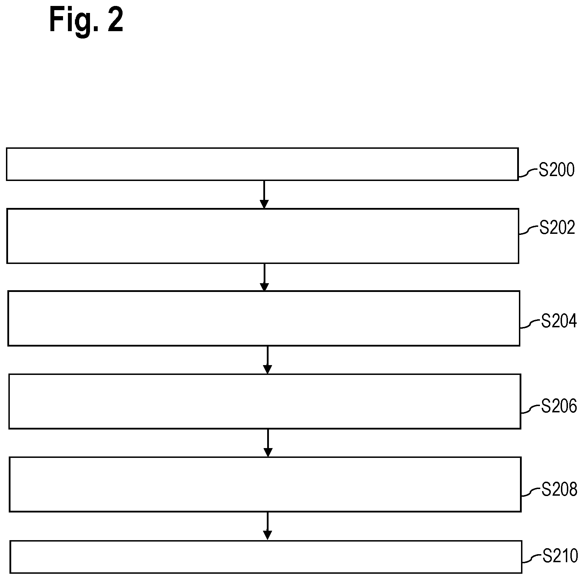

Figure 2 shows a flow chart of a method for generating a marker in a biological sample. In particular, the biological sample is one of a plurality of biological samples. The biological sample comprises a plurality of cells. Preferably, the cells are viable cells, in particular, mammalian cells. For example, the biological sample may be a spheroid.

The method starts in step S200. In step S202, the biological sample or the plurality of biological samples is incubated in the presence of a plurality of at least the oligonucleotide constructs 100 in order to introduce the oligonucleotide constructs 100 into the biological sample. For example, this can be achieved by transfecting the cells of the biological sample with the oligonucleotide construct 100. Particularly suitable methods of transfection are lipofection and electroporation. This results in the oligonucleotide constructs 100 being introduced into the cells of the biological sample, in particular into the cytoplasm of the cells.

In step S204, at least a first region of the biological sample is exposed to or scanned with a first spatially constrained light beam. The first spatially constrained light beam is configured to remove the photoremovable cage molecules 108 from the oligonucleotide construct 100, in particular, only from those oligonucleotide constructs 100 in the first region. For example, the first light beam may have a specific wavelength, which causes the photo-removal of the cage molecule 108. Alternatively or in addition, other parameters of the first light beam may cause the photo-removal of the cage molecule 108, for example, the light intensity or the length of time for which the region is exposed to the light beam. The spatially constrained light beam may be generated and directed by means of an optical device, in particular a fluorescence microscope, a light-sheet microscope, a confocal microscope, or a multiphoton microscope.

In addition, the biological sample may be imaged in step S204 before and/or after the first region is exposed to the first light beam. The image may be generated by the optical device, as well, for example, with a microscope comprising a flow cell. In particular, an image taken before exposing the first region to the first light beam may be used to identify the first region in the biological sample and generate coordinates that describe the position of the first region within the biological sample. The optical device may then expose the first region defined by the coordinates. Moreover, the biological sample may be imaged after exposing the first region with the first light beam.

Once the cage molecules 108 are removed from the oligonucleotides constructs 100 in the first region and respective oligonucleotide constructs 112 are generated, the fluorescent protein regions 104 of oligonucleotide constructs 112 within cells of the biological sample in the first region are expressed. This results in the fluorescent protein 106 being produced within cells of the first region of the biological sample. The first region with the fluorescent proteins 106 may also be called a marker, which marks or labels the biological sample.

In step S206 the biological sample is stored, for example in a liquid in a reservoir for the purpose of cultivation. Alternatively, the biological sample may be processed in experimental analysis or subjected to experimental conditions. During step S206, the biological sample may be stored in the reservoir together with other biological samples of the plurality of biological samples.

In step S208 the biological sample is imaged, for example, by means of the optical device. In addition, in case in step S206 the biological sample is stored in the reservoir together with other biological samples of the plurality of biological samples, each biological sample of the plurality of biological samples may be imaged. Further in step S208 it is determined, whether or not the marker, in particular, the biological sample with the marker, is present in one of the images generated in step S208. This enables identifying or recognising the biological sample with the marker. Further, this enables comparing the image of the biological sample with images of the biological sample that were previously generated.

The method ends in steps S210.

Moreover, the steps S206 and S208 may be iterated. Thus, the biological sample with the marker may be repeatedly stored or processed (S206) and subsequently imaged and identified (S208).

In step S204, at least a second region of the biological sample is exposed to the first light beam. This results in a marker with the first region and the second region expressing the fluorescent protein regions 104 and generating the fluorescent protein 106. This enables generating a more diverse set of possible markers. For example, by varying the distance between the first and second region, several distinct and distinguishable, in particular, unique, markers may be generated. When generating these distinguishable markers in several separate biological samples, this further enables distinguishing between these several biological samples in step S208.

Additionally or alternatively, the plurality of biological samples may be incubated in step S202 with the plurality of oligonucleotide constructs 100 and a plurality of second oligonucleotide constructs in order to introduce the oligonucleotide constructs 100 and the second oligonucleotide constructs into the biological sample. The second oligonucleotide constructs differ from the oligonucleotide constructs 100 by comprising a second fluorescent protein region, which is a nucleic acid sequence encoding a second fluorescent protein, by comprising a second photoremovable cage molecule, and by comprising a second promoter. The fluorescent protein 106 differs from the second fluorescent protein by having different fluorescent properties such as fluorescent excitation wavelength, emission wavelength and/or emission intensity.

The cage molecule 108 and the second cage molecule differ in their conditions under which they are photoremovable from the respective oligonucleotide construct. Specifically, the cage molecule 108 and the second cage molecule may be removed from the respective oligonucleotide construct by light with differing parameters. These parameters include wavelength, intensity and duration of scanning. For example, the cage molecule 108 may be a protecting group removeable by ultraviolet light such as 6-nitropiperonyloxymethyl. Whereas the second cage molecule may be a protecting group removable by a different wavelength, for example, near-infrared light, as described by Vorobev and Moskalensky (c.f. Vorobev AY, Moskalensky AE. Long-wavelength photoremovable protecting groups: On the way to in vivo application. Computational and Structural Biotechnology Journal, 2020, 18, 27-34.). This enables selectively removing the cage molecules 108 or the second cage molecules from the respective plurality of oligonucleotide constructs. In addition, scanning with longer wavelength light, such as near-infrared light, results in less damage of the biological structures scanned by the light beam.

The promoter 102 and the second promoter may comprise the same sequence. Alternatively, the promoter 102 and the second promoter may have different sequences, in particular, the promoters may differ in case the respective oligonucleotide constructs have fluorescent protein regions, which express fluorescent proteins with differences in their respective fluorescence emission intensity, for example. In this case, the promoter activity of an oligonucleotide construct may be matched to the respective fluorescence emission intensity.

Further, in the step S204, at least a second region of the biological sample is then exposed to or scanned with a second spatially constrained light beam. This second light beam is configured to remove the second photoremovable cage molecules from the second oligonucleotide construct, in particular, only from those second oligonucleotide constructs in the second region. As described above, the first and second light beams have different properties in order to selectively remove the cage molecules 108 or the second cage molecules. Thus, in the first region only the oligonucleotide constructs 108 are uncaged and in the second region only the second oligonucleotide constructs are uncaged. Consequently, in the first region only the first fluorescent proteins 106 are generated and in the second region only the second fluorescent proteins are generated. This enables generating an even more diverse set of possible markers. For example, by having regions of different fluorescent colours in addition to varying the distance between the regions, a large number of distinct and distinguishable markers may be generated. This enables distinguishing between a larger number of biological samples in step S208.

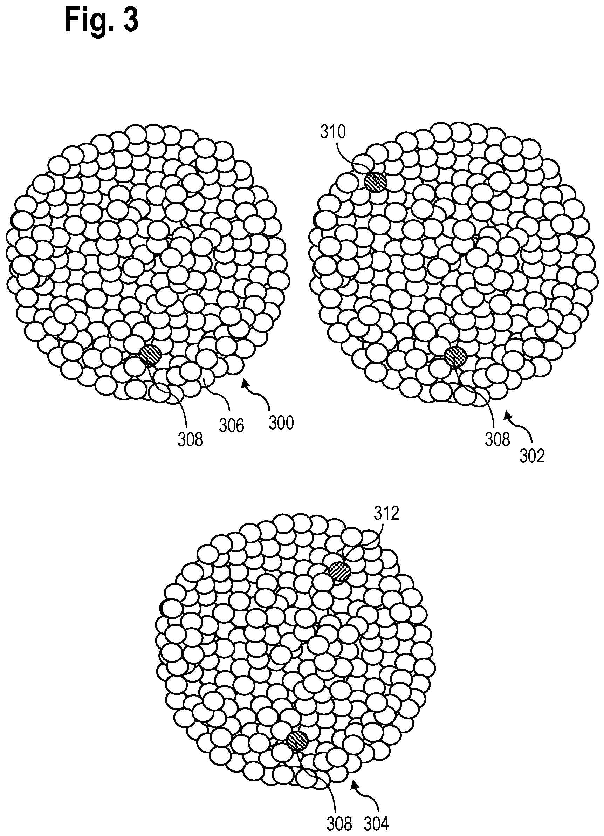

Figure 3 shows by way of example three biological samples 300, 302, 304, each with markers generated by the method described for Figure 2. The biological samples 300, 302, 304 comprise a plurality of cells 306. The biological sample 300 has a marker that comprises a first fluorescent cell 308 generating the first fluorescent protein with a first emission wavelength. The biological sample 302 has a marker that comprises the first fluorescent cell 308 and a second fluorescent cell 310 similarly generating the first fluorescent protein. The biological sample 304 has a marker that comprises the first fluorescent cell 308 and a third fluorescent cell 312 generating the second fluorescent protein at a second emission wavelength.

As an example, the biological samples 300, 302, 304 shown in Figure 3 may be stored in a reservoir together. The unique markers on each biological sample 300, 302, 304 would nevertheless enable identifying and/or distinguishing the biological samples 300, 302, 304 from each other.

For the biological sample 300, 302, 304, the regions exposed to the respective light beams were shaped in the form of the respective cells 308, 310, 312. Alternatively, the regions may form an oval shape, or a rectangular shape on the biological sample. Further, the regions may comprise several cells, such that several adjacent cells may generate the respective fluorescent protein.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100: Oligonucleotide construct
- 102: Promoter region
- 104: Fluorescent protein region
- 106: Fluorescent protein
- 108: Photoremovable cage molecule
- 110: Light
- 112: Uncaged oligonucleotide construct
- 300, 302, 304: Biological sample with marker
- 306: Cell
- 308, 310, 312: Fluorescent cell

## Claims

1. A method for generating a marker in a biological sample (302, 304) comprising a plurality of cells by means of oligonucleotide constructs (100), wherein the biological sample (302, 304) is one of a plurality of biological samples, the method comprising the following steps:
introducing at least a plurality of first oligonucleotide constructs (100) into the biological sample (302, 304), wherein the first oligonucleotide constructs (100) comprise a first promoter (102), a first nucleic acid sequence (104) encoding a first fluorescent protein (106), and a first photoremovable cage molecule (108),
exposing, in particular scanning, at least a first region and a further region of the biological sample with a first spatially constrained, in particular focused, light beam to form uncaged first oligonucleotide constructs (112) in order to enable synthesis of first fluorescent proteins (106) from the first nucleic acid sequence (104) and generate at least a part of the marker in the first region and the further region of the biological sample (302, 304),
wherein, by varying a distance between the first region and the further region, distinct and distinguishable markers are generated in several separate biological samples (302, 304) of the plurality of biological samples, and
imaging the biological sample (302, 304) and determining, whether or not the marker is present in the generated image of the biological sample (302, 304), and identifying the biological sample (302, 304) based on at least the first region and the further region of the generated marker.

2. The method according to claim 1, wherein the biological sample (302, 304) is imaged prior to the exposing step in order to generate an image of the biological sample (302, 304), in particular to identify at least the first region in the image data, and/or
wherein the biological sample (302, 304) is imaged after the exposing step in order to generate an image of the biological sample (302, 304) with the marker.

3. The method according to one of the preceding claims, wherein the step of introducing the oligonucleotide constructs (100) is performed by transfection, in particular by lipofection or electroporation.

4. The method according to one of the preceding claims, wherein a plurality of second oligonucleotide constructs (100) is introduced into the biological sample (302, 304), and wherein the second oligonucleotide constructs (100) comprise a second promoter (102), a second nucleic acid sequence (104) encoding a second fluorescent protein (106), and a second photoremovable cage molecule (108).

5. The method according to claim 4, wherein a second region of the biological sample (302, 304) is exposed with a second spatially constrained, in particular focused, light beam to form uncaged second oligonucleotide constructs (112) in order to enable synthesis of second fluorescent proteins (106) from the second nucleic acid sequence (104) and generate at least a part of the marker in the second region of the biological sample (302, 304).

6. The method according to claim 5, wherein the first spatially constrained light beam and the second spatially constrained light beam differ in at least one light parameter, wherein said at least one light parameter comprises an uncaging wavelength, a predetermined pulse length or a light intensity.

7. The method according to one of the preceding claims 4 to 6, wherein the first fluorescent protein (106) and the second fluorescent protein have different fluorescent emission wavelengths.

8. The method according to one of the preceding claims, wherein the spatially constrained light beams are generated by means of an optical device, in particular, by a widefield fluorescence microscope, an epi-fluorescence microscope, a light-sheet microscope, a confocal microscope, or a multiphoton microscope.

9. The method according to one of the preceding claims, wherein the regions of the biological sample (302, 304) have one of the following shapes: circular, ovoid, linear, or the shape of a single cell of the biological sample (302, 304).

## Patentansprüche

1. Verfahren zur Erzeugung eines Markers in einer biologischen Probe (302, 304), die eine Vielzahl von Zellen umfasst, mittels Oligonukleotidkonstrukten (100), wobei die biologische Probe (302, 304) eine von einer Vielzahl von biologischen Proben ist, wobei das Verfahren die folgenden Schritte umfasst:
Einbringen von mindestens einer Vielzahl von ersten Oligonukleotidkonstrukten (100) in die biologische Probe (302, 304), wobei die ersten Oligonukleotidkonstrukte (100) einen ersten Promotor (102), eine erste Nukleinsäuresequenz (104), die ein erstes fluoreszierendes Protein (106) codiert, und ein erstes photoremovierbares Käfigmolekül (108) umfassen, Belichten, insbesondere Abscannen, von mindestens einem ersten Bereich und
einem weiteren Bereich der biologischen Probe mit einem ersten räumlich begrenzten, insbesondere fokussierten, Lichtstrahl zur Bildung von entkäfigten ersten Oligonukleotidkonstrukten (112), um die Synthese von ersten fluoreszierenden Proteinen (106) aus der ersten Nukleinsäuresequenz (104) zu ermöglichen und mindestens einen Teil des Markers in dem ersten Bereich und dem weiteren Bereich der biologischen Probe (302, 304) zu erzeugen,
wobei durch Variieren eines Abstands zwischen dem ersten Bereich und dem weiteren Bereich verschiedene und unterscheidbare Marker in mehreren getrennten biologischen Proben (302, 304) der Vielzahl von biologischen Proben erzeugt werden, und
Abbilden der biologischen Probe (302, 304) und Bestimmen, ob der Marker in dem erzeugten Bild der biologischen Probe (302, 304) vorhanden ist oder nicht, und Identifizieren der biologischen Probe (302, 304) basierend auf mindestens dem ersten Bereich und dem weiteren Bereich des erzeugten Markers.

2. Verfahren nach Anspruch 1, wobei die biologische Probe (302, 304) vor dem Belichtungsschritt abgebildet wird, um ein Bild der biologischen Probe (302, 304) zu erzeugen, insbesondere um mindestens den ersten Bereich in den Bilddaten zu identifizieren, und/oder
wobei die biologische Probe (302, 304) nach dem Belichtungsschritt abgebildet wird, um ein Bild der biologischen Probe (302, 304) mit dem Marker zu erzeugen.

3. Verfahren nach einem der vorstehenden Ansprüche, wobei der Schritt des Einführens der Oligonukleotidkonstrukte (100) durch Transfektion, insbesondere durch Lipofektion oder Elektroporation, durchgeführt wird.

4. Verfahren nach einem der vorstehenden Ansprüche, wobei eine Vielzahl von zweiten Oligonukleotidkonstrukten (100) in die biologische Probe (302, 304) eingeführt wird, und wobei die zweiten Oligonukleotidkonstrukte (100) einen zweiten Promotor (102), eine zweite Nukleinsäuresequenz (104), die ein zweites fluoreszierendes Protein (106) codiert, und ein zweites photoremovierbares Käfigmolekül (108) umfassen.

5. Verfahren nach Anspruch 4, wobei ein zweiter Bereich der biologischen Probe (302, 304) mit einem zweiten räumlich begrenzten, insbesondere fokussierten, Lichtstrahl belichtet wird, um entkäfigte zweite Oligonukleotidkonstrukte (112) zu bilden, um die Synthese von zweiten fluoreszierenden Proteinen (106) aus der zweiten Nukleinsäuresequenz (104) zu ermöglichen und mindestens einen Teil des Markers in dem zweiten Bereich der biologischen Probe (302, 304) zu erzeugen.

6. Verfahren nach Anspruch 5, wobei der erste räumlich begrenzte Lichtstrahl und der zweite räumlich begrenzte Lichtstrahl sich in mindestens einem Lichtparameter unterscheiden, wobei der mindestens eine Lichtparameter eine Entkäfigungswellenlänge, eine vorbestimmte Pulslänge oder eine Lichtintensität umfasst.

7. Verfahren nach einem der vorstehenden Ansprüche 4 bis 6, wobei das erste fluoreszierende Protein (106) und das zweite fluoreszierende Protein unterschiedliche fluoreszierende Emissionswellenlängen aufweisen.

8. Verfahren nach einem der vorstehenden Ansprüche, wobei die räumlich begrenzten Lichtstrahlen mittels einer optischen Vorrichtung, insbesondere mittels eines Weitfeldfluoreszenzmikroskops, eines Epi-Fluoreszenzmikroskops, eines Lichtblattmikroskops, eines konfokalen Mikroskops oder eines Multiphotonenmikroskops, erzeugt werden.

9. Verfahren nach einem der vorstehenden Ansprüche, wobei die Bereiche der biologischen Probe (302, 304) eine der folgenden Formen aufweisen: kreisförmig, eiförmig, linear oder die Form einer einzelnen Zelle der biologischen Probe (302, 304).

## Revendications

1. Procédé de génération d'un marqueur dans un échantillon biologique (302, 304) comprenant une pluralité de cellules au moyen de constructions oligonucléotidiques (100), dans lequel l'échantillon biologique (302, 304) est l'un d'une pluralité d'échantillons biologiques, le procédé comprenant les étapes suivantes :
introduction d'au moins une pluralité de premiers constructions oligonucléotidiques (100) dans l'échantillon biologique (302, 304), dans lesquels les premiers constructions oligonucléotidiques (100) comprennent un premier promoteur (102), une première séquence d'acide nucléique (104) codant pour une première protéine fluorescente (106), et une première molécule cage photolabile (108),
exposition, en particulier balayage, d'au moins une première région et d'une autre région de l'échantillon biologique avec un premier faisceau lumineux spatialement contraint, en particulier focalisé, pour former des premiers constructions oligonucléotidiques (112) décagés afin de permettre la synthèse de premières protéines fluorescentes (106) à partir de la première séquence d'acide nucléique (104) et de générer au moins une partie du marqueur dans la première région et l'autre région de l'échantillon biologique (302, 304),
dans lequel, en faisant varier une distance entre la première région et l'autre région, des marqueurs distincts et différentiables sont générés dans plusieurs échantillons biologiques (302, 304) séparés de la pluralité d'échantillons biologiques, et
formation d'image de l'échantillon biologique (302, 304) et détermination de la présence ou non du marqueur dans l'image générée de l'échantillon biologique (302, 304), et identification de l'échantillon biologique (302, 304) sur la base d'au moins la première région et de l'autre région du marqueur généré.

2. Procédé selon la revendication 1, dans lequel l'échantillon biologique (302, 304) fait l'objet d'une formation d'image avant l'étape d'exposition afin de générer une image de l'échantillon biologique (302, 304), en particulier pour identifier au moins la première région dans les données d'image, et/ou dans lequel l'échantillon biologique (302, 304) fait l'objet d'une formation d'image après l'étape d'exposition afin de générer une image de l'échantillon biologique (302, 304) avec le marqueur.

3. Procédé selon l'une des revendications précédentes, dans lequel l'étape d'introduction des constructions oligonucléotidiques (100) est réalisée par transfection, en particulier par lipofection ou électroporation.

4. Procédé selon l'une des revendications précédentes, dans lequel une pluralité de seconds constructions oligonucléotidiques (100) est introduite dans l'échantillon biologique (302, 304), et dans lequel les seconds constructions oligonucléotidiques (100) comprennent un second promoteur (102), une seconde séquence d'acide nucléique (104) codant pour une seconde protéine fluorescente (106), et une seconde molécule cage photolabile (108).

5. Procédé selon la revendication 4, dans lequel une seconde région de l'échantillon biologique (302, 304) est exposée à un second faisceau lumineux spatialement contraint, en particulier focalisé, pour former des seconds constructions oligonucléotidiques décagés (112) afin de permettre la synthèse de secondes protéines fluorescentes (106) à partir de la seconde séquence d'acide nucléique (104) et de générer au moins une partie du marqueur dans la seconde région de l'échantillon biologique (302, 304).

6. Procédé selon la revendication 5, dans lequel le premier faisceau lumineux spatialement contraint et le second faisceau lumineux spatialement contraint diffèrent par au moins un paramètre lumineux, dans lequel ledit au moins un paramètre lumineux comprend une longueur d'onde de décaging, une durée d'impulsion prédéterminée ou une intensité lumineuse.

7. Procédé selon l'une des revendications précédentes 4 à 6, dans lequel la première protéine fluorescente (106) et la seconde protéine fluorescente présentent des longueurs d'onde d'émission de fluorescence différentes.

8. Procédé selon l'une des revendications précédentes, dans lequel les faisceaux lumineux spatialement contraints sont générés au moyen d'un dispositif optique, en particulier, par un microscope à fluorescence en champ large, un microscope à épifluorescence, un microscope à nappe de lumière, un microscope confocal, ou un microscope multiphotonique.

9. Procédé selon l'une des revendications précédentes, dans lequel les régions de l'échantillon biologique (302, 304) présentent l'une des formes suivantes : circulaire, ovoïde, linéaire, ou la forme d'une cellule unique de l'échantillon biologique (302, 304).
